# FASCICULE DE BREVET EUROPEEN

(11) **EP 1 842 535 B1**
(45) Date de publication et mention de la délivrance du brevet: **18.08.2010**
(21) Numéro de dépôt: 07290420.4
(22) Date de dépôt: 06.04.2007
(51) Int. Cl.: A61K 31/165, A61P 25/22

(54) **Utilisation de l'agomélatine pour l'obtention de médicaments destinés au traitement du Trouble Anxiété Généralisée**
Verwendung von Agomelatin zur Herstellung von Medikamenten zur Behandlung der generalisierten Angststörung
Use of agomelatine to obtain a medication aimed at treating generalised anxiety disorder

(30) Priorité: 07.04.2006 FR 0603083
(43) Date de publication de la demande: 10.10.2007
(73) Titulaire: Les Laboratoires Servier, 92415 Courbevoie Cedex (FR)
(72) Inventeur: Delalleau, Bruno, 92600 Asnieres-sur-Seine (FR); Fabiano, Agnès, 92410 Ville d'Avray (FR); Millan, Mark, 78230 Le Pecq (FR); Mocaer, Elisabeth, 92200 Neuilly sur Seine (FR)

(56) Documents cités:
- EP-A- 1 564 202
- EP-A1- 0 447 285
- WO-A-2005/002562
- WO-A-2006/096434
- MOCAËR ELISABETH ET AL: "[Development of a new antidepressant : agomelatine]" MÉDECINE SCIENCES : M/S. OCT 2005, vol. 21, no. 10, octobre 2005 (2005-10), pages 888-893, XP009074822 ISSN: 0767-0974
- MONTGOMERY S A ET AL: "Absence of discontinuation symptoms with agomelatine and occurrence of discontinuation symptoms with paroxetine: A randomized, double-blind, placebo-controlled discontinuation study" INTERNATIONAL CLINICAL PSYCHOPHARMACOLOGY, vol. 19, no. 5, septembre 2004 (2004-09), pages 271-280, XP009074840 ISSN: 0268-1315

## Description

La présente invention concerne l'utilisation de l'agomélatine ou *N*-[2-(7-méthoxy-1-naphthyl)éthyl]acétamide de formule (I) : 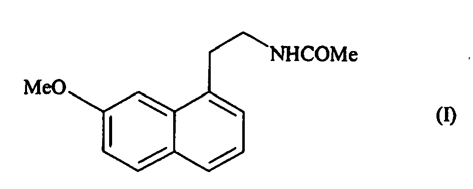 ainsi que ses hydrates, formes cristallines et sels d'addition à un acide ou à une base pharmaceutiquement acceptable, pour l'obtention de médicaments destinés au traitement du Trouble Anxiété Généralisée ou TAG.

L'agomélatine ou *N*-[2-(7-méthoxy-1-naphthyl)éthyl]acétamide présente la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

La demanderesse a présentement découvert que l'agomélatine ou *N*-[2-(7-méthoxy-1-naphthyl)éthyl]acétamide, ainsi que ses hydrates, formes cristallines et sels d'addition à un acide ou à une base pharmaceutiquement acceptable, possédait des propriétés intéressantes permettant de l'utiliser dans le traitement du Trouble Anxiété Généralisée.

Caractérisé par une anxiété importante et sans objet, le Trouble Anxiété Généralisée répond à des critères parfaitement définis et constitue une entité nosographique à part entière (300.02 - DSM IV - Manuel Diagnostique et Statistique des Troubles Mentaux, 4ème édition, American Psychiatric Association). Cette pathologie chronique entraîne de nombreux troubles associés, et notamment des dysfonctionnements cognitifs, plus particulièrement de la pensée et des représentations mentales, des altérations du raisonnement, du jugement et donc des performances, mais entraîne également des troubles psychomoteurs sources de maladresse, de capacités de réaction amoindries, voire anihilées (Wittchen HU et al., Arch. Gen. Psychiatry, 1994, 51 (5), 355-364).

La spécificité de ce trouble est par ailleurs reconnue par les autorités de santé européenne et américaine qui ont éditées des « guidelines » de développement pour les molécules revendiquant spécifiquement cette indication (Committee for Proprietary Medicinal Product (CPMP) /EWP/4284/02/ Londres - 20 Janvier 2005 - Guideline on the clinical investigation for the treatment of médicinal products indicated for Generalized Anxiety Disorder).

Des études épidémiologiques récentes montrent une prévalence pour cette pathologie d'environ 5 à 6 % dans la population générale avec un taux pouvant atteindre 10% pour les femmes de plus de 40 ans. L'impact de cette pathologie en terme de santé et d'économie est donc important.

Il n'existe pas actuellement de traitement réellement satisfaisant et reconnu pour le Trouble Anxiété Généralisée. Les benzodiazépines ont longtemps été et restent dans certains pays le traitement de première intention. L'art antérieur W02006/096434 décrit l'utilisation de la flibansérine en association avec un ou plusieurs anxiolytiques pour le traitement des troubles de l'anxiété. Il est également divulgué dans WO2005/002562 une association contenant un inhibiteur sélectif de la recapture de la sérotonine et de l'agomélatine pour le traitement de la dépression, des troubles de l'anxiété et autres troubles affectifs.

Plus récemment, l'administration de molécules comme la venlafaxine, la paroxétine ou le escitalopram a été préconisée. Cependant ces différentes catégories thérapeutiques sont à l'origine de nombreux effets secondaires, les plus répertoriés étant la sédation, la pharmaco-dépendance, l'interaction avec l'alcool, et un impact cardiovasculaire et/ou sexuel non négligeable...

Par ailleurs, dans la plupart des cas, l'arrêt de ces traitements se traduit par l'apparition d'un syndrome de discontinuation, difficilement acceptable par le patient.

Ainsi la mise à disposition de nouveaux traitements pour cette pathologie est une nécessité comme le souligne la « guideline » CPMP.

L'agomélatine, nouvelle entité chimique aux propriétés pharmacologiques novatrices, a montré lors d'études cliniques contrôlées versus placebo une efficacité dans l'épisode dépressif majeur.

La Demanderesse a présentement découvert que l'agomélatine, de par ses propriétés pharmacologiques, pouvait être utilisé dans le Trouble Anxiété Généralisée.

Une étude clinique réalisée versus placebo sur des patients souffrant du Trouble Anxiété Généralisée a permis de montrer de façon significative que l'agomélatine constituait un traitement tout à fait adapté à cette pathologie.

De plus, outre l'activité observée dans le traitement du Trouble Anxiété Généralisée, l'agomélatine présente un bon profil d'acceptabilité auprès des patients : en particulier, l'agomélatine est dépourvu des effets secondaires associés aux psychotropes classiques. Parmi ces effets, le syndrome de discontinuation observé à l'arrêt du traitement avec les psychotropes classiques est inexistant avec l'agomélatine, ce qui en fait un traitement de choix dans cette pathologie.

L'invention concerne donc l'utilisation de l'agomélatine, ainsi que ses hydrates, formes cristallines et sels d'addition à un acide ou à une base pharmaceutiquement acceptable, pour l'obtention de compositions pharmaceutiques destinées au traitement du Trouble Anxiété Généralisée.

Particulièrement, l'invention concerne l'utilisation de l'agomélatine obtenue sous la forme cristalline II décrite dans la demande de brevet EP 1 564 202, pour l'obtention de compositions pharmaceutiques destinées au traitement du Trouble Anxiété Généralisée.

Les compositions pharmaceutiques seront présentées sous des formes convenant aux administrations par voie orale, parentérale, transcutanée, nasale, rectale, perlinguale, et notamment sous forme de préparations injectables, comprimés, comprimés sublinguaux, glossettes, gélules, capsules, tablettes, suppositoires, crèmes, pommades, gels dermiques, etc..,

Outre l'agomélatine, les compositions pharmaceutiques selon l'invention contiennent un ou plusieurs excipients ou véhicules choisis parmi des diluants, dés lubrifiants, des liants, des agents de désintégration, des absorbants, des colorants, des édulcorants, etc...

A titre d'exemple et de manière non limitative, on peut citer :
◆ *pour les diluants :* le lactose, le dextrose, le sucrose, le mannitol, le sorbitol, la cellulose, la glycérine,
◆ *pour les lubrifiants :* la silice, le talc, l'acide stéarique et ses sels de magnésium et de calcium, le polyéthylène glycol,
◆ *pour les liants :* le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

La posologie utile varie selon le sexe, l'âge et le poids du patient, la voie d'administration, la nature de l'affection et des traitements éventuellement associés et s'échelonne entre 1 mg et 50 mg d'agomélatine par 24 heures.

De manière préférentielle, la dose journalière d'agomélatine sera de 25 mg par jour, avec possibilité d'augmenter à 50 mg par jour.

### Composition pharmaceutique :

| Formule de préparation pour 1000 comprimés dosés à 25 mg : | |
|---|---|
| N-[2-(7-méthoxy-1-naphthyl)éthyl]acétamide | 25 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

### Etude clinique :

Une étude clinique versus placebo a été réalisée sur 121 patients. Ces 121 patients ont été randomisés en deux groupes parallèles et ont reçu soit de l'agomélatine à 25 mg par jour, soit du placebo.
Après deux semaines de traitement et en cas de réponse insuffisante, la dose a été augmentée en double aveugle à 50 mg pour les patients sous agomélatine par un système IVRS (Interactive Voice Response System). Les patients ont été traités douze semaines.

L'efficacité a été appréciée à l'aide d'instruments d'évaluation recommandés par les autorités de santé, notamment l'échelle d'Anxiété de Hamilton (Hamilton M., J. Neurol. Neurosurg. Psychiat., 1959, 23, 56-62), ou l'échelle de retentissement fonctionnel de Sheehan (International Clinical Psychopharmacology, 1996, 11, 89-95). Le profil d'acceptabilité a également été évalué.

Les résultats obtenus montrent que la différence sur le score total de l'échelle de Hamilton, critère principal d'évaluation, entre le groupe sous traitement avec l'agomélatine et le groupe sous placebo est de -3,28 (p=0,040), ce qui correspond à une différence cliniquement et statistiquement significative.

L'étude montre également un bon profil d'acceptabilité du produit par les patients, ainsi que l'absence de syndrome de discontinuation à l'arrêt du traitement.

## Revendications

1. Utilisation de l'agomélatine ou *N*-[2-(7-méthoxy-1-naphthyl)éthyl]acétamide ou un de ses hydrates, formes cristallines et sels d'addition à un acide ou à une base pharmaceutiquement acceptable, comme seul principe actif pour l'obtention d'un médicament destiné au traitement du Trouble Anxiété Généralisée.

2. Utilisation selon la revendication 1 **caractérisée en ce que** l'agomélatine est obtenue sous la forme cristalline II.

3. Compositions pharmaceutiques contenant comme seul principe actif de l'agomélatine ou un de ses hydrates, formes cristallines et sels d'addition à un acide ou à une base pharmaceutiquement acceptable, seul ou en combinaison avec un ou plusieurs excipients pharmaceutiquement acceptables pour utilisation dans le traitement du Trouble Anxiété Généralisée.

4. Composition pharmaceutique selon la revendication 3 **caractérisée en ce que** l'agomélatine est obtenue sous la forme cristalline II.

## Claims

1. Use of agomelatine, or *N*-[2-(7-methoxy-1-naphthyl)ethyl]acetamide, or one of its hydrates, crystalline forms and addition salts with a pharmaceutically acceptable acid or base, as sole active ingredient, in obtaining a medicament intended for the treatment of generalised anxiety disorder.

2. Use according to claim 1, **characterised in that** the agomelatine is obtained in the crystalline form II.

3. Pharmaceutical compositions comprising, as sole active ingredient, agomelatine, or one of its hydrates, crystalline forms and addition salts with a pharmaceutically acceptable acid or base, alone or in combination with one or more pharmaceutically acceptable excipients, for use in the treatment of generalised anxiety disorder.

4. Pharmaceutical composition according to claim 3, **characterised in that** the agomelatine is obtained in the crystalline form II.

## Patentansprüche

1. Verwendung von Agomelatin oder *N*-[2-(7-Methoxy-1-naphthyl)-ethyl]-acetamid oder seiner Hydrate, Kristallformen und Additionssalze mit einer pharmazeutisch annehmbaren Säure oder Base als alleinigen Wirkstoff für die Herstellung eines Arzneimittels zur Behandlung von generalisierten Angststörungen.

2. Verwendung nach Anspruch 1, **dadurch gekennzeichnet, dass** Agomelatin in der Kristallform II vorliegt.

3. Pharmazeutische Zubereitung enthaltend als alleinigen Wirkstoff Agomelatin oder eines seiner Hydrate, Kristallformen und Additionssalze mit einer pharmazeutisch annehmbaren Säure oder Base allein oder in Kombination mit einem oder mehreren pharmazeutisch annehmbaren Hilfsstoffen zur Verwendung bei der Behandlung von generalisierten Angststörungen.

4. Pharmazeutische Zubereitung nach Anspruch 3, **dadurch gekennzeichnet, dass** Agomelatin in der Kristallform II vorliegt.
